# EUROPEAN PATENT SPECIFICATION

(11) **EP 2 032 031 B1**
(45) Date of publication and mention of the grant of the patent: **17.08.2011**
(21) Application number: 07719118.7
(22) Date of filing: 27.06.2007
(51) Int. Cl.: A61B 5/07, G01N 23/20, G01N 33/50

(54) **Detection of an abnormal change in the component of a hair sample in subjects suffering from breast cancer**
Nachweis einer anormalen Veränderung in der Komponente einer Haarprobe von Patienten mit Brustkrebs
Détection de changement anormal à un composant dans un échantillon de cheveux prélevé chez des sujets présentant un cancer du sein

(30) Priority: 29.06.2006 AU 2006903508; 19.01.2007 AU 2007900272; 22.01.2007 AU 2007900308
(43) Date of publication of application: 11.03.2009
(73) Proprietor: SBC Research Pty Ltd, Sydney, New South Wales 2000 (AU)
(72) Inventor: CORINO, Gary, L., Sydney, NSW 2000 (AU)
(74) Representative: Poulsen, Niels Jakob
(86) International application number: PCT/AU2007/000879
(87) International publication number: WO 2008/000020

(56) References cited:
- WO-A1-00/11469
- WO-A1-00/34774
- WO-A2-03/060513
- CN-A- 1 847 846
- US-A1- 2006 024 705
- US-B1- 6 350 582
- JAMES V ET AL: "EARLY DIAGNOSIS OF BREAST CANCER BY HAIR DIFFRACTION" INTERNATIONAL JOURNAL OF CANCER, JOHN WILEY & SONS, INC, UNITED STATES, SWITZERLAND, GERMANY, vol. 114, no. 6, 11 January 2005 (2005-01-11), pages 969-972, XP007900980 ISSN: 0020-7136
- CORINO GL & FRENCH PW: "Diagnosis of breast cancer by X-ray diffraction of hair" INTERNATIONAL JOURNAL OF CANCER, vol. 122, 23 October 2007 (2007-10-23), pages 847-856, XP002567867
- JAMES VD: "A place for fiber diffraction in the detection of breast cancer?" CANCER DETECTION AND PREVENTION, vol. 30, no. 3, 26 July 2006 (2006-07-26), pages 233-238, XP0024900484 DOI: doi:10.1016/j.cdp.2006.04.001
- LYMAN D. ET AL.: 'Fourier Transform Infrared attenuated total reflection analysis of human hair: Comparison of hair from breast cancer patients with hair from healthy subjects' APPLIED SPECTROSCOP. vol. 59, no. 1, 2005, pages 26 - 32, XP008100668
- AKSIROV A. ET AL.: 'Biological and medical application of SR from the storage rings of VEPP-3 and "Siberia-2". The origin of specific changes of small X rax diffraction pattern of hair and their correlation with the elemental content' NUCLEAR INSTRUMENTS AND METHODS IN PHYSICS RESEARCH A vol. 470, 2001, pages 380 - 387, XP004303843
- KIST A. ET AL.: 'Mapping of ecologically unfavourable territories based on human hair composition' BIOLOGICAL TRACE ELEMENT RESEARCH vol. 64, 1998, pages 1 - 12, XP008102018
- FEUGHELMAN M. ET AL.: 'Hexagonal Packing of Intermediate Filaments (Microfibrils) in alpha-Keratin Fibers' TEXTILE RESEARCH J. vol. 68, no. 2, 1998, pages 110 - 114, XP008103565
- TOWLER M. ET AL.: 'Raman spectroscopy of the human nail: A potential tool for evaluating bone health' J. MATER. SCI.: MATER. MED. vol. 18, 2007, pages 759 - 763, XP019503691

## Description

### BACKGROUND

A variety of research to date has indicated the presence of abnormal changes in components of keratin samples, such as hair, taken from subjects, who are afflicted with a pathological state which can include cancer.

X-ray diffraction analysis has shown that subjects with a plurality of certain types of cancers (colon, breast and prostate) and other pathological states (Alzheimer's disease) produce hair samples that have abnormalities in them. The abnormalities are detectable using X-ray diffraction techniques and are consistent with the presence of the pathological state itself.

The international patent application WO 00/34774 discloses a method for detecting various conditions including breast cancer, wherein a hair sample is washed in distilled water, dried and exposed to fibre X-ray diffraction. The changes in the ultrastructure of the hair are detected and compared with a control.

Whilst X-ray diffraction techniques can identify the presence of an abnormality in hair or other keratin samples, X-ray diffraction techniques are limited in that they do not reveal the nature of the abnormal component (i.e. whether the abnormality represents an inclusion of a chemical substance not normally present or rather a defect in the structure of a keratin fibril for example in the hair). A structural abnormality therefore can have the same proportion of chemical molecules present but differ structurally in geometric orientation of the molecules or their chemical bonding. Therefore, the pathological state can lead to structural changes in chemical bonding in the hair even though the chemical molecules forming the hair are in the same proportion as occurs in normal hair.

A variety of different causes as to the nature of chemical abnormalities present in the hair of persons with a pathological state have been conjectured. It has been suggested that an increase in specific metals normally found in hair may be associated with various pathological states.

Some researchers have conjectured that different types of growth factors can be found in increased proportions in the hair of cancer patients. Other researchers have suggested that rather than specific additions to normal hair such as increases in the content of certain metals present, that abnormal structures, for example a truncated form of keratin, can be present in hair taken from persons with certain pathological states such as breast cancer.

Irrespective of the truth of the above conjectures the structure of hair, contains a tightly bound cuticle surrounding a dense cortex so that it is often extremely difficult to chemically penetrate hair in an attempt to identify the presence of an abnormal component.

If the abnormal component whether structural or an atypical inclusion in hair taken from a subject with a pathological state, can be isolated, then the pathological state can be identified with greater specificity and sensitivity. Identification of the abnormal component means that additional information is then provided about the nature of the pathological state.

It is therefore seen that a problem sought to be solved by the present invention is the ability to chemically penetrate a hair sample so as to detect an abnormal component within the hair sample taken from a subject with breast cancers.

It is an object of the present invention to address or at least ameliorate some of the above disadvantages.

### Notes

1. The term "comprising" (and grammatical variations thereof) is used in this specification in the inclusive sense of "having" or "including", and not in the exclusive sense of "consisting only of".

### BRIEF DESCRIPTION OF INVENTION

In one broad form of the invention there is provided a method for detecting a presence of an acid-extractable Synchrotron Small Angle X-ray Scatter (SAXS) ring-producing component in a hair sample taken from a subject to determine whether or not the subject has breast cancer comprising the steps of:
a) exposing the hair sample to an organic acid so as to penetrate the hair sample thereby producing a derived biological substance;
b) obtaining data from the derived biological substance;
c) comparing the data obtained from the derived biological substance with a second group of data contained in a reference database so as to identify the presence of the acid-extractable SAXS ring-producing component in the hair sample;
whereby detection of the acid-extractable SAXS ring-producing component is consistent with a presence of breast cancer in the subject.

Preferably, the organic acid includes formic acid.

Preferably, the second group of data is correlated with the presence of breast cancer in the subject.

Preferably, the second group of data is causatively associated with the presence of breast cancer in the subject.

Preferably, the organic accid is selected from a plurality of different organic acids.

Preferably, the hair sample is selected from a plurality of different hair samples.

Preferably, the second group of data is selected from a plurality of different groups of data.

Preferably, the derived data and the second group of data are analyzed using a plurality of different methods of comparison.

Preferably, in use, the hair sample can be obtained and analyzed in association with at least one of a pharmacy, a test kit, the subject's home, a health care clinic and a testing laboratory or any convenient location (office, field or barn).

Preferably, the subject is selected from the group consisting of a human, and a mammal.

### BRIEF DESCRIPTION OF DRAWINGS

Figure 1 shows the application of a swelling substance to a hair sample for use in diagnostic testing.
Figure 2 shows a plurality of different chemical substances being applied in the alternative to a hair sample.
Figure 3 shows a plurality of different hair samples taken from different subjects with different pathological states (or the same subject with several pathological states) or from a plurality of different mammalian species.
Figure 4 shows a plurality of different methods of analysis being used to analyze data produced according to the first embodiment disclosed in Figure 1.
Figure 5 shows the method of analyzing a hair sample being implemented in use, wherein a sample can be collected from a subject at a health care clinic, a collecting room or using a kit at a convenient location to the subject (home, field, barn, etc).
Figure 6 illustrates results of SAXS analysis applied to samples treated in accordance with embodiments of the method of the present invention.

### DETAILED DESCRIPTION OF PREFERRED EMBODIMENTS

Embodiments of the present invention will now be described with reference to the accompanying drawings wherein:

### Definitions:

The plurality of different selections and forms pertaining to the invention as claimed include the selections and forms as appearing in the body of the specification. "Mammalian species" includes the types of species as appearing in the body of the specification. It can include a human, a pet such as a dog or cat or a variety of other animals with hair.

The hair sample 16 is human scalp or body hair and in particular pubic hair, pet hair, animal hair or hair from a member of a mammalian species in general.

A "subject" includes a mammalian species. A mammalian species can include a human, a pet such as a dog or cat or a variety of other animals.

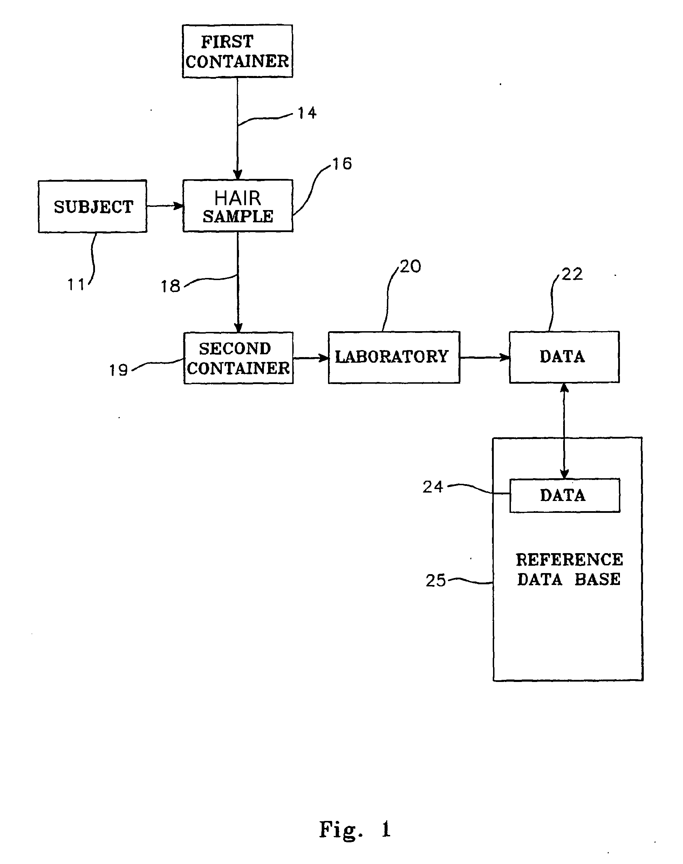

Unless otherwise indicated by the context, a claim to one element is consistent with a claim to at least one element. Figure 1 illustrates a method of analyzing a hair sample 16. Figure 1 shows a first container 12 which holds a swelling substance 14. A hair sample 16 is taken from subject 11, wherein the subject 11 can include a mammalian species. A mammalian species can include a human, a pet such as a dog or cat or a variety of other animals. The hair sample 16 is human scalp or body hair and in particular pubic hair, pet hair, animal hair or hair from a mammalian species in general.

The hair sample 16 is exposed to the swelling substance 14. A derived chemical substance 18 is obtained from application of the swelling substance 14 to the hair sample 16 whereby the swelling substance 14 penetrates the hair sample 16. The derived chemical substance 18 is collected in the second container 19 (alternatively and without loss of generality only one container need be used).

The derived chemical substance 18 located in the second container 19 is then taken to a laboratory 20 at the place of application of the swelling substance 14. The laboratory 20 will then use a plurality of diagnostic tests to obtain data 22 which can be compared with data 24 located in a reference database 25 so as to determine if the subject 11 has breast cancer. The reference database 25 can be obtained from a plurality of control samples taken from normal subjects and subjects with a plurality of different pathological states.

The data 22 can be compared with data 24 in the reference database 25 to determine whether or not the subject 11 has breast cancer, for example if the reference database 25 indicates that the result in question is both correlated and causatively linked to breast cancer then a meaningful comparison can be considered. Additionally zero correlation or no information being provided in the case of no association with an abnormal component being present in the derived chemical substance 18 can in certain instances be consistent with an abnormality being a defect in protein folding, that is a structural defect in the hair sample 16 as opposed to an additional inclusion of a component that may or may not be ordinarily found in the hair sample 16 such as a metal or other compound (whereby no inclusion is washed out into the derived chemical substance 18).

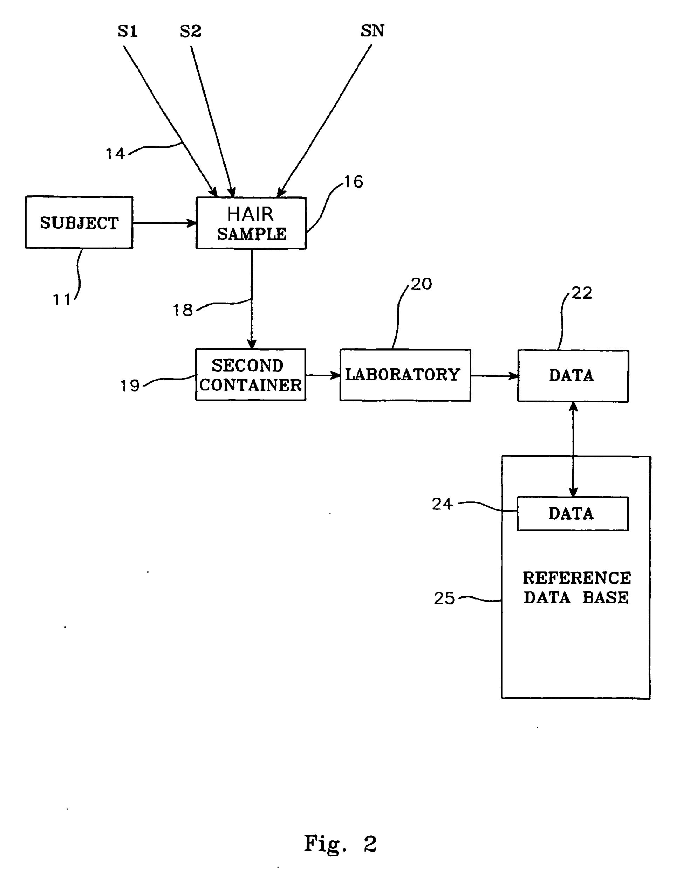

Figure 2 shows an embodiment of the present invention in which the sensitivity or specificity of the method described in Figure 1 is improved by way of changing the swelling substance 14. Figure 2 shows a plurality of different swelling substances 14, being S1, S2...SN.

In Figure 2 the data 22, not shown, is analyzed by comparing the data 22 with the data 24 (from a reference database 25 as seen in Figure 1) so as to select a swelling substance from the set S1...SN, which is adapted to remove hair the defect in the hair sample 16 by either altering the structural nature of the hair sample 16 so as to restore normal folding and configuration of the hair sample 16 or by transferring, by way of application of the swelling substance 14 to the hair sample 16, the component causing the abnormality in the hair sample 16 to the derived chemical substance 18 (this can involve for example formic acid penetrating and washing out an inclusion from a hair sample). Whilst formic acid has been disclosed herein a number of other substances are amenable to penetrate hair so as to remove an abnormal component; other organic acids include acetic acid, propionic acid, butyric acid, trifluoracetic acid, or monochloracetic acid. However, unlike formic acid other considerations are relevant such as toxicity and in the case of reducing agents the fact that the structure of the hair sample 16 can be substantially destroyed by the application of the reducing agent. In contrast formic acid is a swelling substance 14 which may not destroy the structure of a hair sample 16 after application.

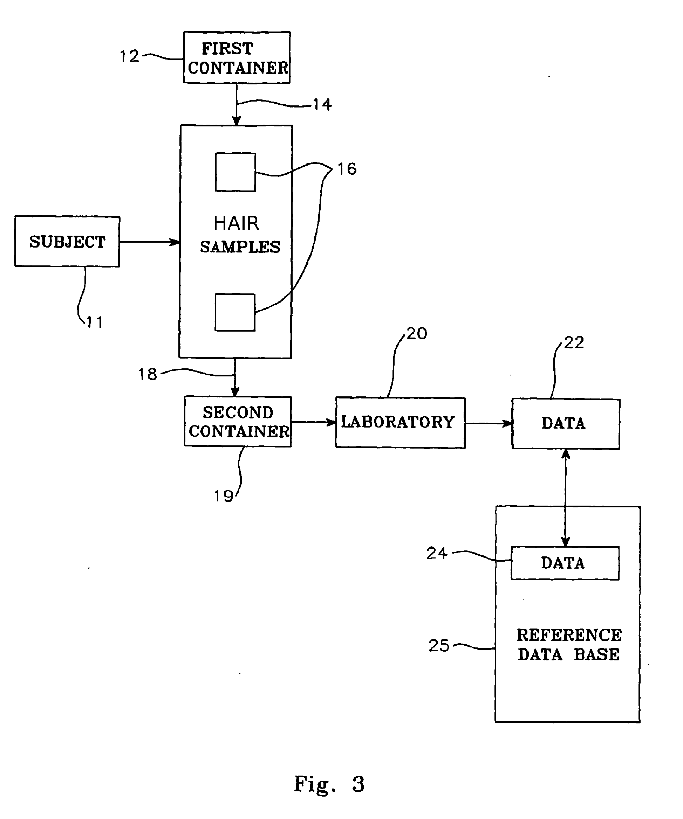

Figure 3 shows the use of a specific swelling substance 14, for a given mode of operation in association with a plurality of different hair samples 16 to yield a derived chemical substance 18. The plurality of different hair samples 16 can be taken from a plurality of different mammalian species. Alternatively or additionally, the plurality of different hair samples 16 can be taken from subjects 11 who are suspected to have a plurality of different pathological states.

The variations in swelling substances as disclosed in Figure 2 and the variations in hair samples 16 as disclosed in Figure 3 (two are shown but more can be envisaged) can be used to identify a particular capability in a particular swelling substance 14 or a particular susceptibility in a given hair sample 16. For example if a given swelling substance 14 such as formic acid was known to remove an abnormal component from a hair sample 16 during a particular concentration range and a particular sub-range of the concentration was shown to yield a significantly improved result then a new availability in the formic acid could exist.

Similarly, as seen in Figure 3, for a given swelling substance 14, having a particular concentration, a systematic variation in the types of hair samples 16 could disclose a susceptibility of a particular type of hair sample 16 to a swelling substance 14 such as formic acid.

A particular susceptibility will occur if a hair sample 16 is known to yield a conclusive result in association with a given mode of operation and a given swelling substance 14 that leads to an improvement in specificity or sensitivity of a diagnostic test to look for the presence of the abnormal component.

The plurality of different keratin samples 16, shown in Figure 3 can include a hair sample 16 taken from a subject 11 who is suspected of having breast cancer.

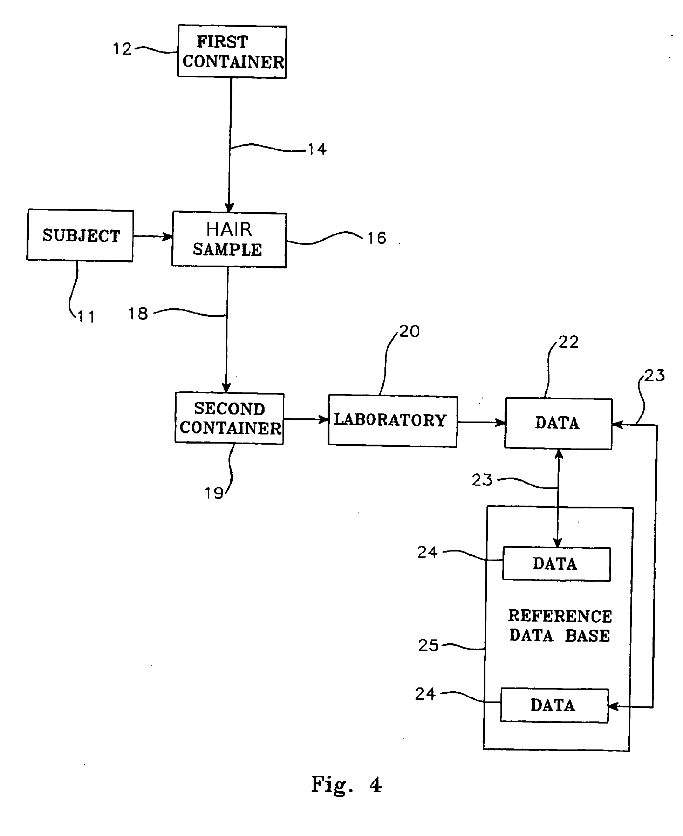

Figure 4 shows an embodiment of the method for detecting the presence of breast cancer in which a given swelling substance 14 and a given hair sample 16 are used in association with a plurality of different types of methods of comparison between the data 22 and a second group of data 24 contained in the reference database 25 so as to produce an improvement in specificity or sensitivity of the method of analyzing the hair sample 16. The plurality of different comparison's 23 shown in Figure 4 (two are shown but more can be envisaged) can without limitation include variations in the mode of operation of the method of analyzing a keratin sample including spectral analysis or the use of pattern recognition computer programs.

An advantage of the present embodiment is that if the abnormal component is present in the derived chemical substance 18 then the derived chemical substance 18 can be further analyzed for the purpose of identifying the nature of the abnormal component so as to provide a health care practitioner with more information about the pathological state. If the abnormal component is a trace metal then atomic absorption spectroscopy or ICP-mass spectrometry could be applied to the derived chemical substance 18 to confirm the nature of the abnormal component. If the abnormal component in the derived chemical substance 18 is a protein, a carbohydrate, a fatty acid or more generally of organic origin and an antibody can be raised against the abnormal component then a plurality of different techniques such as Western blot analysis, ELISA or cell agglutination assays can be used in an attempt to characterize the abnormal component. In certain instances, immuno-electron microscopy can be used in an attempt to identify the abnormal component in the keratin sample 16 without the need for the use of the derived chemical substance 18 to subsequently be obtained. If the abnormal component is associated with genetic material (being hereditary in nature or acquired by way of a viral vector) then an amplification technique could be applied to determine the sequence and conformation of the abnormal component.

### In use

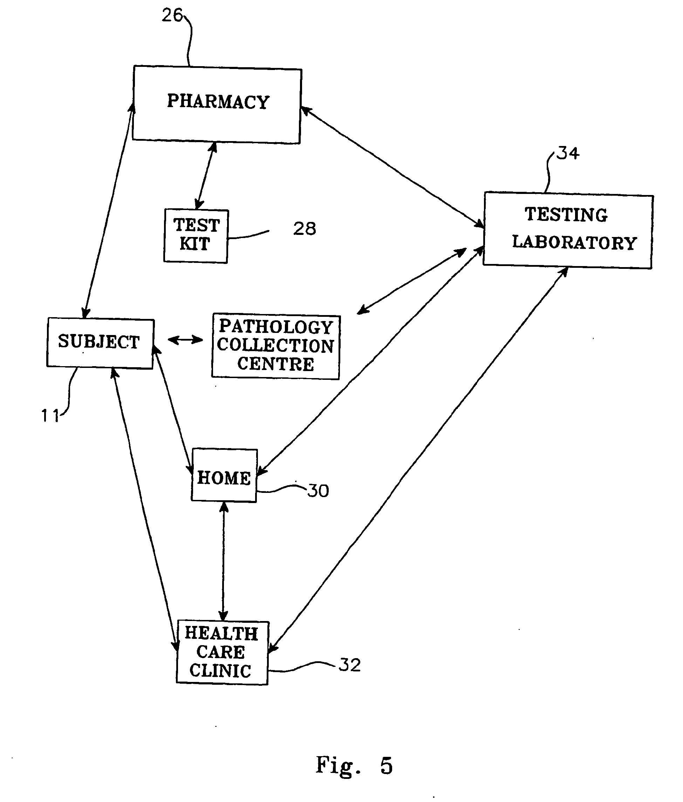

Figure 5 shows an embodiment of the present invention in use. In Figure 5 a hair sample 16 can be collected from a subject at a pharmacy 26 . The hair sample 16 can then be sent to a testing laboratory 34 so as to perform the method for detecting the presence of an abnormal component in the hair sample 16 as seen in Figure 1.

A test kit 28 can be obtained so as to use the test kit 28 embodying the method of detection at the subject's home 30, in association with consultation of the subject's health care practitioner located at a health care clinic 32.

Alternatively, without the need of a home test the subject 11 can visit his or her health care clinic 32 so as to provide the hair sample 16. The health care clinic 32 can perform the method of analyzing the hair sample 16 themselves or in a further, preferred embodiment the health care clinic 32 can obtain the hair sample 16 from the subject 11 so as to forward the hair sample 16 to the testing laboratory 34.

### Further Embodiment

### Organic Acid Treatment of Hair Fibers

It is known that immersion of hair fibers in organic acids such as formic acid and acetic acid can cause them to swell in diameter by as much as 50% and this effect can be reversed by rinsing in water. Blackburn and Lowther (Blackburn S and Lowther AG. The action of organic acids on some fibrous proteins: the oxidation of wool keratin. Biochem J. 1951:49:554-9) reported that a small amount of protein could be extracted from wool fibers by treatment in either formic or acetic acid at room temperature. This methodology was applied to several hair fibers from individuals of known pathology to determine if the ring observed by Synchrotron Small Angle X-Ray Scatter (SAXS) of hair, specific for the presence of breast cancer, could be removed by such treatment. Hair fibers from subjects with breast cancer were immersed in an 85% (v/v) solution of formic acid or in glacial acetic acid for 3 minutes at room temperature. The acid was then decanted and replaced with several changes of Milli-Q water. The fibers were allowed to dry then mounted into a sample holder for exposure to an X-ray source. SAXS image data was collected for these hairs.

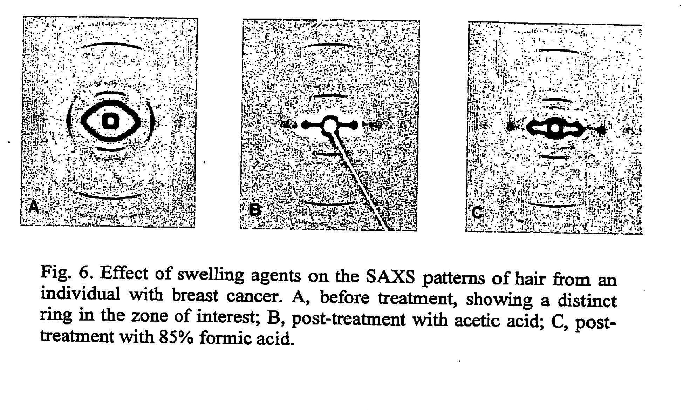

Hair fibers that were taken from the same individual and subsequently treated with formic and acetic acid showed either significant reduction or complete removal of the ring in the zone of interest in the SAXS image. A typical example is demonstrated in Figure 6. Figure 6A is a SAXS image of a hair from an individual with breast cancer. Figures 6B and 6C are SAXS images of hair fibers from the same individual post treatment with acetic acid and formic acid respectively. It can be seen from these images that the ring in the zone of interest is significantly diminished after treatment with either acid and hence such treatments may be used as a tool to investigate the underlying change in the fiber associated with the presence of breast cancer.

## Claims

1. A method for detecting a presence of an acid- extractable Synchrotron Small Angle X-ray Scatter (SAXS) ring-producing component in a hair sample taken from a subject to determine whether or not the subject has breast cancer comprising the steps of:
a) exposing the hair sample to an organic acid so as to penetrate the hair sample thereby producing a derived biological substance;
b) obtaining data from the derived biological substance;
c) comparing the data obtained from the derived biological substance with a second group of data contained in a reference database so as to identify the presence of the acid-extractable SAXS ring-producing component in the hair sample;
whereby detection of the acid-extractable SAXS ring-producing component is consistent with a presence of breast cancer in the subject.

2. The method for detecting the presence of an acid-extractable SAXS ring-producing component in a hair sample as recited in claim 1 wherein the organic acid is formic acid.

3. The method for detecting the presence of an acid-extractable SAXS ring-producing component in a hair sample as recited in any one of claims 1-2 wherein the second group of data is correlated with the presence of breast cancer in the subject.

4. The method for detecting the presence of an acid-extractable SAXS ring-producing component in a hair sample as recited in any one of claims 1-3 wherein the second group of data is causatively associated with the presence of breast cancer in the subject.

5. The method for detecting the presence of an acid-extractable SAXS ring-producing component in a hair sample as recited in any one of claims 1-4 wherein the organic acid is selected from a plurality of different organic acids.

6. The method for detecting the presence of an acid-extractable SAXS ring-producing component in a hair sample as recited in any one of claims 1-5 wherein the hair sample is selected from a plurality of different hair samples.

7. The method for detecting the presence of an acid-extractable SAXS ring-producing component in a hair sample as recited in any one of claims 1-6 wherein the second group of data is selected from a plurality of different data groups of data.

8. The method for detecting the presence of an acid-extractable SAXS ring-producing component in a hair sample as recited in any one of claims 1-7 wherein the derived data and the second group of data are analyzed using a plurality of different methods of comparison.

9. The method for detecting the presence of an acid-extractable SAXS ring-producing component in a hair sample as recited in any one of claims 1-8 wherein, in use, the hair sample can be obtained and analyzed in association with at least one of a pharmacy, a test kit, the subject's home, a health care clinic and a testing laboratory.

10. The method for detecting the presence of an acid-extractable SAXS ring-producing component in a hair sample according to any one of claims 1-9 wherein the subject is selected from the group consisting of a human, and a mammal.

## Patentansprüche

1. Verfahren zum Nachweis der Gegenwart eines mit Säure extrahierbaren, einen SAXS-Ring (Kleinwinkel-Synchrotonröntgenstrahlstreuung) erzeugenden Bestandteils einer Haarprobe eines Patienten um festzustellen, ob der Patient Brustkrebs hat oder nicht, umfassend die Schritte:
a) Aussetzen der Haarprobe an eine organische Sauce zum Eindringen in die Haarprobe, wobei eine abgeleitete biologische Substanz erzeugt wird;
b) Erhalten von Daten über die abgeleitete biologische Substanz;
c) Vergleiche der Daten, die mit der abgeleiteten biologischen Substanz erhalten wurden, mit einer zweiten Gruppe Daten in einer Vergleichsdatenbank zum Nachweis der Gegenwart des mit Säure extrahierbaren, einen SAXS-Ring erzeugenden Bestandteils in der Haarprobe;
wobei der Nachweis des mit Säure extrahierbaren, einen SAXS-Ring erzeugenden Bestandteils mit der Gegenwart von Brustkrebs beim Patienten konform ist.

2. Verfahren zum Nachweis der Gegenwart eines mit Säure extrahierbaren, einen SAXS-Ring erzeugenden Bestandteils in einer Haarprobe nach Anspruch 1, wobei die organische Säure Ameisensäure ist.

3. Verfahren zum Nachweis der Gegenwart eines mit Säure extrahierbaren, einen SAXS-Ring erzeugenden Bestandteils in einer Haarprobe nach einem der Ansprüche 1-2, wobei die zweite Gruppe Daten mit der Gegenwart von Brustkrebs bei dem Patienten korreliert.

4. Verfahren zum Nachweis der Gegenwart eines mit Säure extrahierbaren, einen SAXS-Ring erzeugenden Bestsandteils in einer Haarprobe nach einem der Ansprüche zur wobei die zweite Gruppe Daten ursächlich mit der Gegenwart von Brustkrebs bei dem Patienten zusammenhängt.

5. Verfahren zum Nachweis der Gegenwart eines mit Säure extrahierbaren, einen SAXS-Ring erzeugenden Bestandteils in einer Haarprobe nach einem der Ansprüche 1-4, wobei die organische Säure, ausgewählt ist aus einer Mehrzahl unterschiedlicher organischer Säuren.

6. Verfahren zum Nachweis der Gegenwart eines mit Säure extrahierbaren, einen SAXS-Ring erzeugenden Bestsandteils in einer Haarprobe nach des Ansprüche 1-5, wobei die Hauptprobe ausgewählt ist aus einer Mehrzahl unterschiedlicher Haarproben.

7. Verfahren zum Nachweis der Gegenwart eines mit Saure extrahierbaren, einen SAXS-Ring erzeugenden Bestandteils in einer Haarprobe nach einem der Ansprüche 1-6, wobei die zweite Gruppe Daten ausgewählt ist aus einer Mehrzahl unterschiedlicher Datengruppen Daten.

8. Verfahren zum Nachweis der Gegenwart eines mit Säure extrahierbaren, einen SAXS-Ring erzeugenden Bestandteils in einer Haarprobe nach einem der Ansprüche 1-7, wobei die abgeleiteten Daten und die zweite Gruppe Daten unter Verwendung einer Mehrzahl unterschiedlicher Vergleichsverfahren analysiert werden.

9. Verfahren zum Nachweis der Gegenwart eines mit Säure extrahierbaren, einen SAXS-Ring erzeugenden Bestsandteils in einer Haarprobe nach des Ansprüche 1-8, wobei die Haarprobe bei der Verwendung in Verbindung mit mindestens einer Apotheke, einem Testsatz, dem Zuhause des Patienten, einem Versorgungsstandort und einem Prüflabor genommen und analysiert werden kann.

10. Verfahren zum Nachweis der Gegenwart eines mit Säure extrahierbaren, einen SAXS-Ring erzeugenden Bestandteils in einer Haarprobe nach einem der Ansprüche 1-9, wobei der Patient ausgewählt ist aus der Gruppe, bestehend aus einem Menschen und einem Säugetier.

## Revendications

1. Méthode de détection de la présence d'un composant extractible à l'acide produisant un anneau de diffusion aux petits angles des rayons X de synchrotron (SAXS) dans un échantillon de cheveux prélevé sur un sujet afin de déterminer si le sujet est atteint ou non de cancer du sein comprenant les étapes suivantes :
a) exposition de l'échantillon de cheveux à un acide organique afin qu'il pénètre dans l'échantillon de cheveux et produise ainsi une substance biologique dérivée;
b) obtention de données à partir de la substance biologique dérivée ;
c) comparaison des données obtenues à partir de la substance biologique dérivée avec un deuxième groupe de données contenu dans une base de données de préférence de manière à identifier la présence du composant extractible à l'acide produisant un anneau SAXS dans l'échantillon de cheveux ;
par laquelle la détection du composant extractible à l'acide produisant un anneau SAXS concorde avec la présence d'un cancer du sein chez le sujet,

2. La méthode de détection de la présence d'un composant extractible à l'acide produisant un anneau SAXS dans un échantillon de cheveux telle qu'exposée dans la revendication 1 dans laquelle l'acide organique est l'acide formique,

3. La méthode de détection de la présence d'un composant extractible à l'acide produisant un anneau SAXS dans un échantillon de cheveux telle qu'exposée dans l'une quelconque des revendications 1 à 2 dans laquelle le deuxième groupe de données est corrélé avec la présence d'un cancer du sein chez le sujet.

4. La méthode de détection de la présence d'un composant extractible à l'acide produisant un anneau SAXS dans un échantillon de cheveux telle qu'exposée dans l'une quelconque des revendications 1 à 3 dans laquelle le deuxième groupe de données est associé de manière causative à la présence d'un cancer du sein chez le sujet.

5. La méthode de détection de la présence d'un composant extractible à l'acide produisant un anneau SAXS dans un échantillon de cheveux telle qu'exposée dans l'une quelconque des revendications 1 à 4 dans laquelle l'acide organique est choisi parmi plusieurs acides organiques différents,

6. La méthode de détection de la présence d'un composant extractible à l'acide produisant un anneau SAXS dans un échantillon de cheveux telle qu'exposée dans l'une quelconque des revendications 1 à 5 dans laquelle l'échantillon de cheveux est choisi parmi plusieurs échantillons de cheveux différents.

7. La méthode de détection de la présence d'un composant extractible à l'acide produisant un anneau SAXS dans un échantillon de cheveux telle qu'exposée dans l'une quelconque des revendications 1 à 6 dans laquelle le deuxième groupe de données est choisi parmi plusieurs groupes de données différents,

8. La méthode de détection de la présence d'un composant extractible à l'acide produisant un anneau SAXS dans un échantillon de cheveux telle qu'exposée dans l'une quelconque des revendications 1 à 7 dans laquelle les données dérivées et le deuxième groupe de données sont analysés en utilisant plusieurs méthodes de comparaison différentes.

9. La méthode de détection de la présence d'un composant extractible à l'acide produisant un anneau SAXS dans un échantillon de cheveux telle qu'exposée dans l'une quelconque des revendications 1 à 8 dans laquelle, en cours d'utilisation, l'échantillon de cheveux peut être obtenu et analysé en association avec au moins l'un de : une pharmacie, un kit d'essai, le domicile du sujet, un centre de soins et un laboratoire d'essai.

10. La méthode de détection de la présence d'un composant extractible à l'acide produisant un anneau SAXS dans un échantillon de cheveux telle qu'exposée dans l'une quelconque des revendications 1 à 9 dans laquelle le sujet est choisi dans le groupe consistant en un être humain et un mammifère,
